# EUROPEAN PATENT SPECIFICATION

(11) **EP 1 707 106 B1**
(45) Date of publication and mention of the grant of the patent: **20.07.2011**
(21) Application number: 06006426.8
(22) Date of filing: 28.03.2006
(51) Int. Cl.: A61B 1/05

(54) **Electronic endoscope apparatus**
Elektronisches Endoskop
Endoscope électronique

(30) Priority: 28.03.2005 JP 2005090685
(43) Date of publication of application: 04.10.2006
(73) Proprietor: FUJIFILM Corporation, Tokyo (JP)
(72) Inventor: Abe, Kazunori, Kita-ku Saitama-shi, Saitama (JP)
(74) Representative: Klunker . Schmitt-Nilson . Hirsch

(56) References cited:
- WO-A-2004/100395
- JP-A- 2001 046 334
- JP-A- 2004 113 805
- US-A1- 2001 055 061

## Description

### BACKGROUND OF THE INVENTION

### 1. Field of the Invention

The present invention relates to an electronic endoscope apparatus constituted of an electronic endoscope and a processor between which a signal is transmitted and received via radio waves.

### 2. DESCRIPTION OF THE RELATED ARTS

Conventionally, medical diagnoses using electronic endoscopes are widely performed. In the electronic endoscope, an imaging sensor such as a CCD is incorporated in a front end portion of an insertion section for being inserted into a body cavity. Image signals obtained by the CCD is subject to signal processing in a processor to observe an image of the body cavity, that is, an endoscopic image, on a monitor.

The conventional electronic endoscope and the processor are connected through a signal cable. However, a wireless electronic endoscope apparatus is devised which transmits and receives the signals via radio waves without using the signal cable to improve operability of the electronic endoscope (see Japanese Patent Laid-Open Publication No. 2001-251612). In this reference, a modulating section for modulating the signals and a transmitting section for transmitting the signals via the radio waves are provided in the electronic endoscope, and a receiving section for receiving the radio waves and a demodulating section for demodulating the radio waves into the original signals are provided in the processor.

The conventional electronic endoscope with the signal cable requires approximately 4kV of a dielectric strength voltage between a patient circuit in the electronic endoscope and a secondary circuit in the processor. However, such high dielectric strength voltage is unnecessary in the wireless electronic endoscope apparatus since the signal cable is not used between the electronic endoscope and the processor.

Commonly, in a hospital, an endoscope examination room is separated into plural examination rooms by partitions, and the electronic endoscope apparatus is disposed in each of the examination rooms to perform the endoscopic diagnoses to plural patients at the same time. When plural wireless electronic endoscope apparatuses are used, it is necessary to avoid radio interference between the wireless electronic endoscope apparatuses. Further, since a frequency band allocated to the wireless signal transmission/reception between medical devices is limited to a certain range, the signals should be transmitted and received by a system corresponding to the above frequency band. However, the wireless electronic endoscope apparatus disclosed in the above reference uses a TV channel frequency band which is not the particular frequency band for the medical devices. Further, the above wireless electronic endoscope apparatus does not have a means to prevent the radio interference in the signal transmission/reception when plural wireless electronic endoscope apparatuses are used.

In US 2001/055061 A1 and JP 2004-113805 wireless electronic endoscope apparatuses are disclosed in which several endoscopes communicate with a single central receiver.

JP 2004-113805 discloses an electronic endoscope apparatus constituted of an electronic endoscope and a processor, the electronic endoscope including a first channel detection section and a transmission frequency band switching section, the processor including a second channel detection section for detecting an available channel from plural channels previously allocated to each of plural reception frequency bands and a reception frequency band switching section, wherein the first channel detection section transmits a channel-in-use notification signal to said second channel detection section and said second channel detection section transmits a channel number notification signal to said first channel detection section.

### SUMMARY OF THE INVENTION

A main object of the present invention is to provide an electronic endoscope apparatus capable of securely preventing radio interference between the electronic endoscope apparatuses.

Another object of the invention is to provide an electronic endoscope apparatus having improved operability.

In order to achieve the above and other objects, an electronic endoscope apparatus of the present invention is constituted according to claim 1.

The channel allocation request signal is transmitted immediately after the power of the electronic endoscope is turned on. The channel-in-use notification signal is transmitted at constant intervals.

Further, the channel allocation request signal, the channel-in-use notification signal and the channel number notification signal are transmitted and received through a different channel from those allocated to each of the transmission and reception frequency bands.

According to the electronic endoscope apparatus of the present invention, the electronic endoscope includes the first channel detection section for detecting the available channel from the plural channels previously allocated to each of the plural transmission frequency bands. The processor includes the second channel detection section for detecting the available channel from the plural channels previously allocated to each of the plural reception frequency bands. The transmission frequency band switching section of the electronic endoscope automatically switches between the transmission frequency bands according to the detection result of the first channel detection section. The reception frequency band switching section automatically switches between the reception frequency bands according to the detection result of the second channel detection section. Accordingly, the radio interference between the electronic endoscope apparatuses is securely prevented. Further, since it is no longer necessary to manually set the channels and the frequency bands, the operability of the electronic endoscope apparatus is improved.

### BRIEF DESCRIPTION OF THE DRAWINGS

The above and other subjects and advantages of the present invention will become apparent from the following detailed description of the preferred embodiments when read in association with the accompanying drawings, which are given by way of illustration only and thus are not limiting the present invention. In the drawings, like reference numerals designate like or corresponding parts throughout the several views, and wherein:
FIG. 1 is a schematic view illustrating a structure of an electronic endoscope apparatus of the present invention;
FIG. 2 is a block diagram illustrating a structure of an electronic endoscope;
FIG. 3 is a block diagram illustrating a structure of a transmitting section of the electronic endoscope;
FIG. 4 is a block diagram illustrating a structure of a processor;
FIG. 5 is a block diagram illustrating a structure of a receiving section in the processor;
FIG. 6 is an explanatory view illustrating five electronic endoscope apparatuses disposed in five examination rooms separated by partitions;
FIG. 7 is a flow chart illustrating steps for allocation of an available channel in the electronic endoscope; and
FIG. 8 is a flow chart illustrating steps for allocation of the available channel to the electronic endoscope in the processor.

### DETAILED DESCRIPTION OF THE PREFERRED EMBODIMENTS

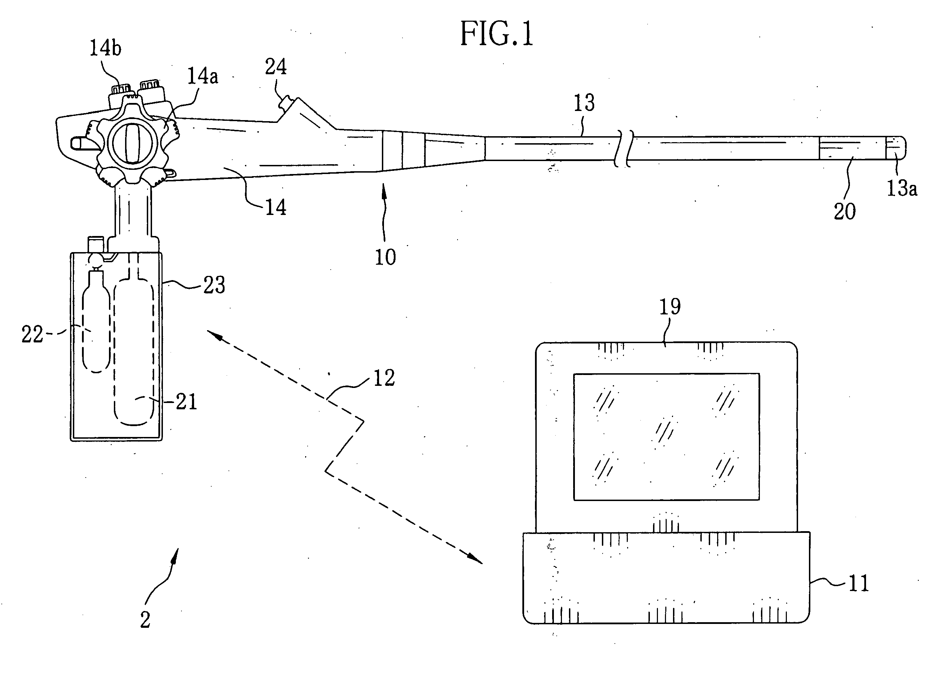

In FIG.1, an electronic endoscope apparatus 2 is constituted of an electronic endoscope 10 and a processor 11. The electronic endoscope 10 and the processor 11 of the electronic endoscope apparatus 2 transmit and receive signals via radio waves within a first or a second frequency bands (for instance, 1.2GHz or 2.4GHz). To each of the first and second frequency bands, plural channels are previously allocated.

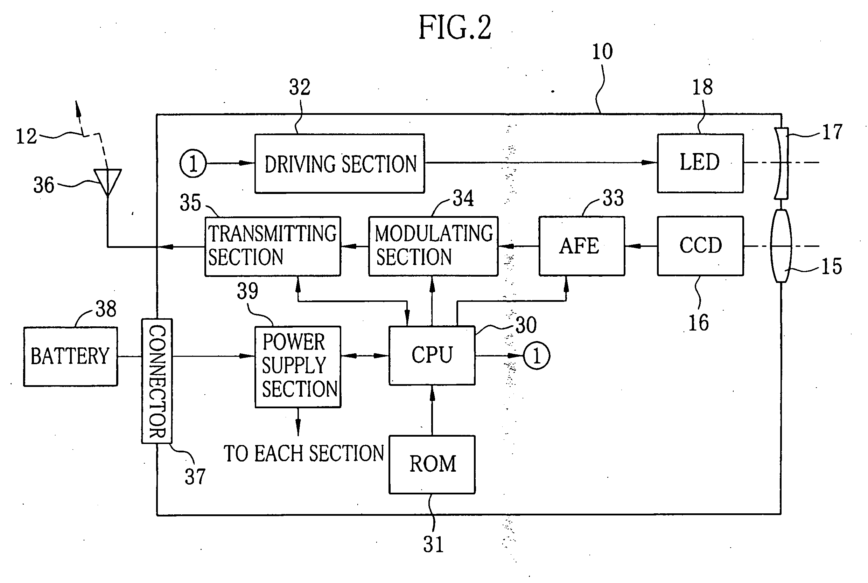

The electronic endoscope 10 is provided with an insertion section 13 inserted into a body cavity, and an operating section 14 connected to a base end portion of the insertion section 13. A front end section 13a at a front end portion of the insertion section 13 incorporates an objective lens 15 for taking in image light of an observation area in the body cavity, a CCD 16 which is an image sensor for capturing the image of the observation area in the body cavity, and an illumination lens 17 and an LED light source (hereinafter, an LED) 18 for illuminating the body cavity (see Fig. 2). The image of the body cavity taken by the CCD 16 is displayed as an endoscopic image on amonitor 19 connected to the processor 11.

Behind the front end section 13a, there is a flexible section 20 formed of plural joint pieces . Awire extending through the insertion section 13 is pushed and pulled by operating an angle knob 14a provided in the operating section 14 to bend the flexible section 20 in the up, down, right and left directions. Thus, the front end section 13a can be directed toward a desired direction inside the body cavity.

A cartridge 23 incorporating a water tank 21 and an air cylinder 22 is attached to a bottom portion of the operating section 14 in a removable manner. Water is stored in the water tank 21 and air is stored in the air cylinder 22. When a water/air supply button 14b in the operating section 14 is operated, the water in the water tank 21 and the air in the air cylinder 22 are respectively supplied through a water pipe and an air pipe and ejected from a washing nozzle (not shown) formed in the front end section 13a to an objective lens 15. Thereby, dirt adhered to a surface of the objective lens 15 is removed and the air is supplied to the body cavity. Since the cartridge 23 is attached to a position where a part of a hand of an operator contacts the cartridge 23 when the operator grips the electronic endoscope 10, the cartridge 23 helps to ensure a solid operation of the electronic endoscope 10. Note that a numeral 24 is a forceps opening into which a treatment tool is inserted.

In FIG. 2, a CPU 30 controls overall operation of the electronic endoscope 10. The CPU 30 is connected to a ROM 31 which stores various programs and data for controlling the operation of the electronic endoscope 10. The CPU 30 reads necessary program and/or data from the ROM 31 to control the operation of the electronic endoscope 10.

A driving section 32 is connected to the LED 18. The driving section 32 drives the LED 18 under control of the CPU 30. The light emitted from the LED 18 illuminates the observation area in the body cavity through the illumination lens 17. Note that it is also possible to dispose the LED 18 inside the operating section 14 instead of the front end section 13a. In this case, the light emitted from the LED light source 18 is introduced to the front end section 13a through a light guide.

The CCD 16 focuses image light of the observation area entered through the objective lens 15 onto its image capture surface, and outputs the image signal corresponding to each pixel to an AFE 33. The AFE 33 performs correlated double sampling, amplification and A/D conversion to the image signal to convert the image signal into a digital image signal, and outputs the digital image signal to a modulating section 34.

The modulating section 34 performs, for instance , a digital quadrature modulation to the digital image signal to generate an RF signal. A transmitting section 35 transmits the RF signal to the processor 11 as a radio wave 12 within a first or a second frequency band through an antenna 36, which will be described later.

A battery 38 is connected to a connector 37. Power of the battery 38 is supplied to each section of the electronic endoscope 10 through a power supply section 39 controlled by the CPU 30. Behind the operating section 14 , a battery chamber (not shown) is provided for accommodating the battery 38, and the connector 37 is disposed inside the battery chamber.

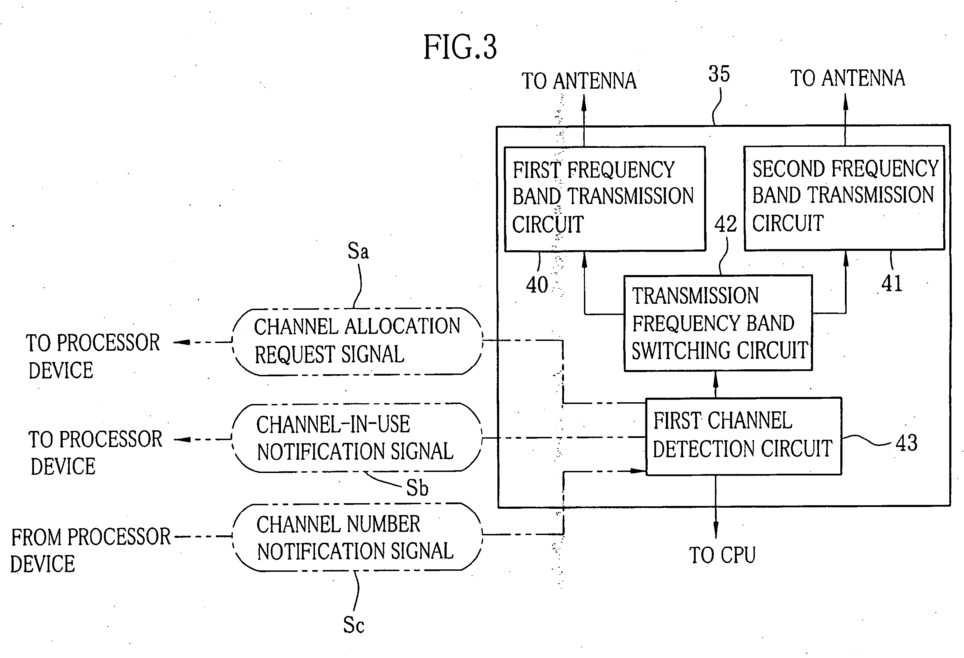

In FIG. 3, in the transmitting section 35 , a first frequency band transmission circuit 40, a second frequency band transmission circuit 41, a transmission frequency band switching circuit 42 and a first channel detection circuit 43 are provided. Each of the first and second frequency band transmission circuits 40, 41 is constituted of a power amplifier, an RF switch, and a circulator. The power amplifier amplifies the RF signal generated in the modulating section 34 to a level receivable by the processor 11. The RF-switch is turned on/off according to a timing of a burst in a TDMA (Time Division Multiple Access) method used in the electronic endoscope apparatus 2. The circulator supplies the RF signal as the radio wave 12 within the first or second frequency band to the antenna 36.

The transmission frequency band switching circuit 42 is connected to a first frequency band transmission circuit 40 and a second frequency band transmission circuit 41. The transmission frequency band switching circuit 42 switches between the first and second frequency band transmission circuits 40, 41 according to a detection result of the first channel detection circuit 43. In an initial setting, the second frequency band transmission circuit 41 is selected by the transmission frequency band switching circuit 42.

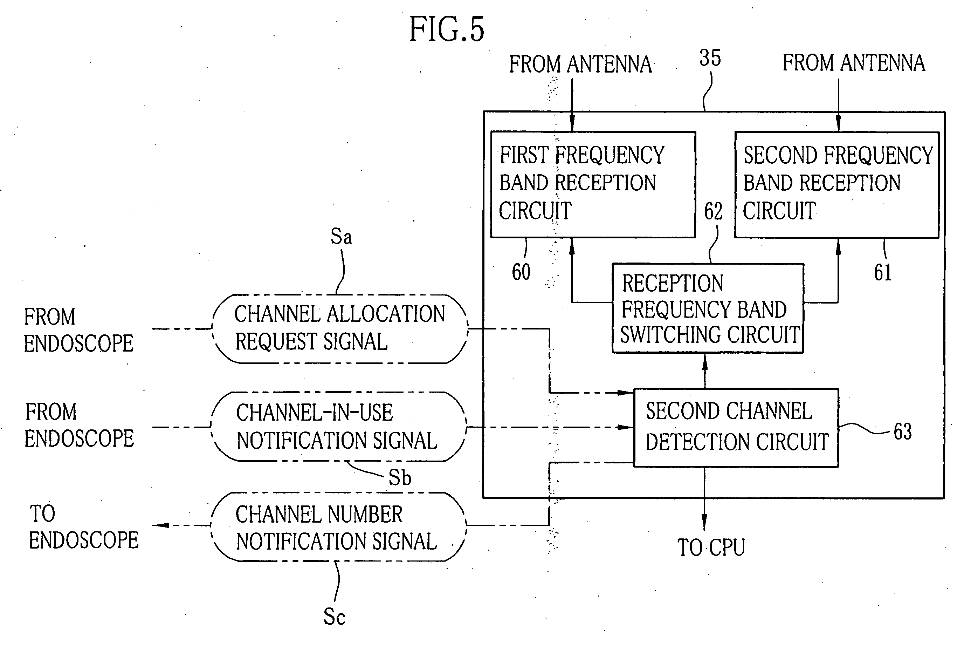

When the power of the electronic endoscope 10 is turned on, the first channel detection circuit 43 transmits a channel allocation request signal Sa to a second channel detection circuit 63 (see FIG. 5) of the processor 11 for requesting a channel allocation. Further, the first channel detection circuit 43 transmits a channel-in-use notification signal Sb to the second channel detection circuit 63 to notify the allocated channel number currently used by the electronic endoscope 10. Further, the first channel detection circuit 43 receives a channel number notification signal Sc notifying an available channel number from the second channel detection circuit 63. The first channel detection circuit 43 transmits the notified available channel number to the CPU 30 and the transmission frequency band switching circuit 42.

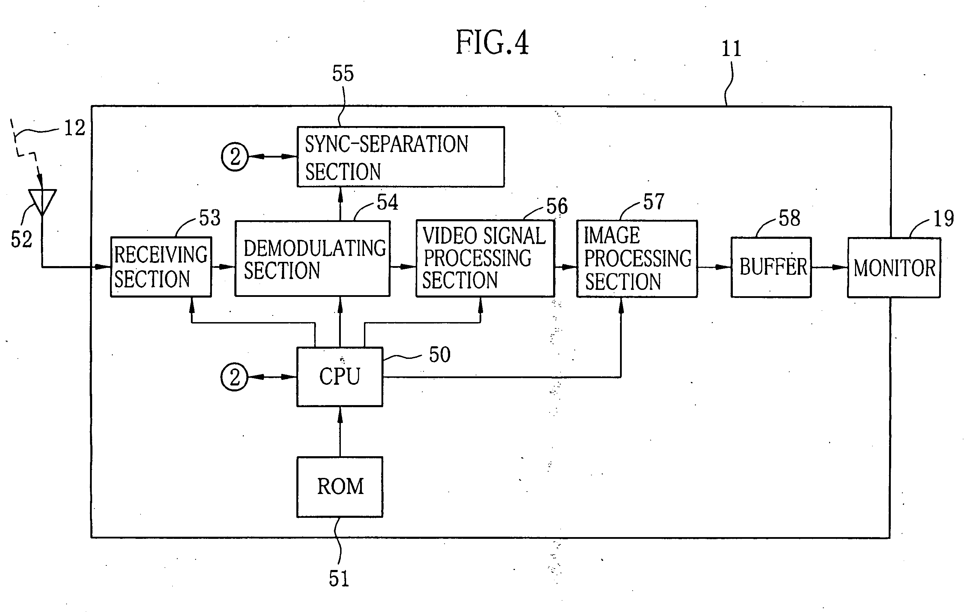

In Fig. 4, a CPU 50 controls overall operation of the processor 11. The CPU 50 is connected to a ROM 51 in which various programs and data for controlling the operation of the CPU 50 are stored. The CPU 50 reads the necessary program and data from the ROM 51 to control the operation of the processor 11.

An antenna 52 receives the radio wave 12 from the electronic endoscope 10. A receiving section 53, as will be described later, amplifies the radio wave 12, that is, the RF signal received through the antenna 52. The demodulating section 54 demodulates the RF signal into the original image signal by, for instance, the digital quadrature detection.

A synch-separation section 55 separates a synchronizing signal from the demodulated image signal by amplitude separation under the control of the CPU 50. Thereafter, a horizontal synchronizing signal and a vertical synchronizing signal are separated from the synchronizing signal by frequency separation. A video signal processing section 56 generates a digital video signal from the image signal. An image processing section 57 performs image processing such as mask generation and addition of character information to the digital video signal. A buffer 58 temporarily stores the digital video signal which will be displayed on the monitor 19 as the endoscopic image.

In Fig. 5, in the receiving section 53, a first frequency band reception circuit 60, a second frequency band reception circuit 61, a reception frequency band switching circuit 62 and the second channel detection circuit 63 are provided. Each of the first and second frequency band reception circuits 60, 61 is constituted of a circulator for supplying the radio waves received through the antenna 52, an RF switch, and a low-noise amplifier for amplifying the RF signals.

The reception frequency band switching circuit 62 is connected to the first and second frequency band reception circuits 60, 61. According to the detection results of the second channel detection circuit 63, the reception frequency band switching circuit 62 automatically switches whether the first and second frequency band reception circuits 60, 61 to be used. In an initial setting, as with the electronic endoscope 10, the second frequency band reception circuit 61 is selected by the reception frequency band switching circuit 62.

Upon receiving the channel allocation request signal Sa from the first channel detection circuit 43, the second channel detection circuit 63 transmits the channel number notification signal Sc to the first channel detection circuit 43 to notify the available channel number according to a channel usage condition notified by the channel-in-use notification signal (s) Sb from currently used endoscope (s) . The second channel detection circuit 63 transmits the channel number notification signal Sc to the CPU 50 and the reception frequency band switching circuit 62. When there is no available channel at the moment, the second channel detection circuit 63 does not transmit the channel number notification signal Sc. Note that the above signals Sa to Sc are transmitted and received as the radio waves 12 between the first and second channel detection circuits 43, 63 through a different channel (0 channel) from those allocated to the first and second frequency bands. In particular, the channel-in-use notification signal Sb is transmitted from the first channel detection circuit 43 at constant intervals.

To observe the observation area in the body cavity by using the electronic endoscope apparatus 2 configured as above, the insertion section 13 is inserted into the body cavity and the LED 18 is turned on to illuminate the body cavity. The endoscopic image obtained by the CCD 16 is observed on the monitor 19.

At this time, the image light of the observation area in the body cavity entered through the objective lens 15 is focused on the image capture surface of the CCD 16, and thereby the image signal is output from the CCD 16 to the AFE 33 . In the AFE 33, the correlated double sampling, the amplification and the A/D conversion are performed to the image signal to convert the image signal into the digital image signal.

In the modulating section 34, the digital quadrature modulation is performed to the digital image signal output from the AFE 33 to generate the RF signal. The RF signal is amplified in the transmitting section 35 and transmitted to the processor 11 as the radio wave 12 through the antenna 36 of the electronic endoscope 10.

When the processor 11 receives the radio wave 12 through the antenna 52, the received radio wave 12, that is, the RF signal is amplified in the receiving section 53. In the demodulating section 54, the digital quadrature detection is performed to the amplified RF signal to demodulate the RF signal and recover the original image signal generated in the electronic endoscope 10.

The sync-separation is performed to the recovered image signal in the sync-separation section 55 under control of the CPU 50. Thereafter, the image signal is output from the video signal processing section 56 as a digital video signal. The output video signal is subject to various image processing in the image processing section 57, temporarily stored in the buffer 58, and displayed on the monitor 19 as the endoscopic image. Thus, the data of the endoscopic image is transmitted and received via the radio wave 12 between the electronic endoscope 10 and the processor 11.

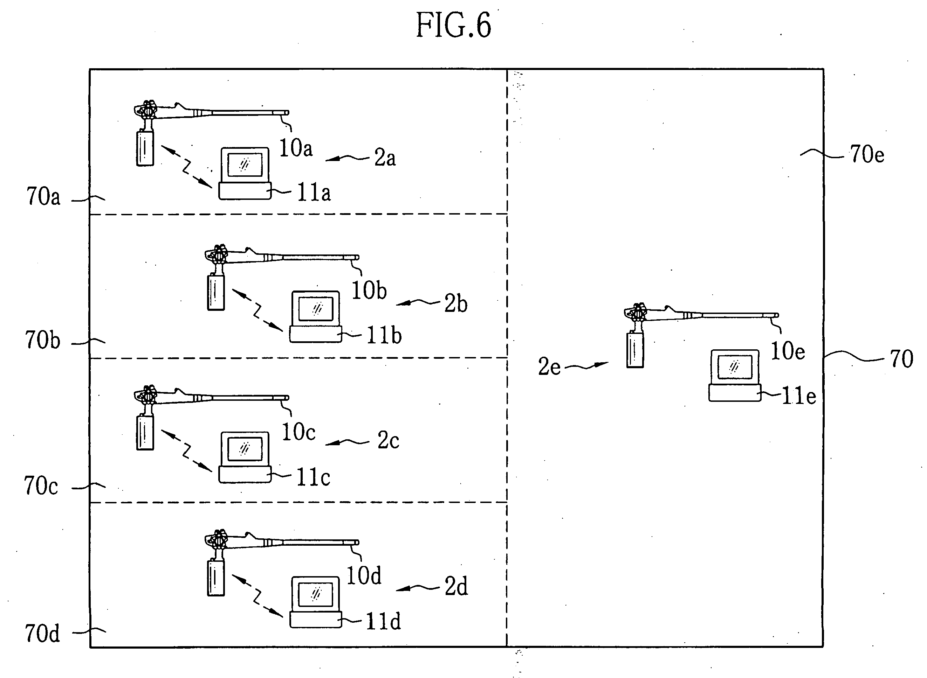

Hereinafter, referring to Figs. 6-8, the process of the channel allocation in the electronic endoscope apparatus 2 having the above configuration is explained. Note that in this example, five channels are allocated to each of the first and second frequency band transmission circuits 40, 41 and the first and second frequency band reception circuits 61, 61. However, the number of the channels is not limited to the above and can be changed according to the available frequency band and/or the available channel bandwidth. Further, since a frequency used in one of the first and second frequency bands is a multiplied frequency to that in the other frequency band, the electronic endoscope 10 or the processor 11 enables to transmit and receive the radio waves 12 in plural frequency bands only by using each single antenna 36 or 52.

As shown in Fig. 6, an endoscope examination room 70 is separated into plural examination rooms 70a-70e by partitions or the like. In the examination rooms 70a-70e, electronic endoscope apparatuses 2a-2e are disposed. The electronic endoscope apparatuses 2a-2e are respectively constituted of electronic endoscopes 10a-10e and processors 11a-11e. The channels one-four of the second frequency band are currently used by the electronic endoscope apparatuses 2a-2d. In this state, the electronic endoscope 10e is wirelessly connected to the processor 11e to perform the endoscopic diagnosis. Note that in the following description, for convenience of distinction, suffixes a-e corresponding to the electronic endoscope apparatus 2a-2e are added to the numerals of parts in the electronic endoscope apparatuses 2a-2e.

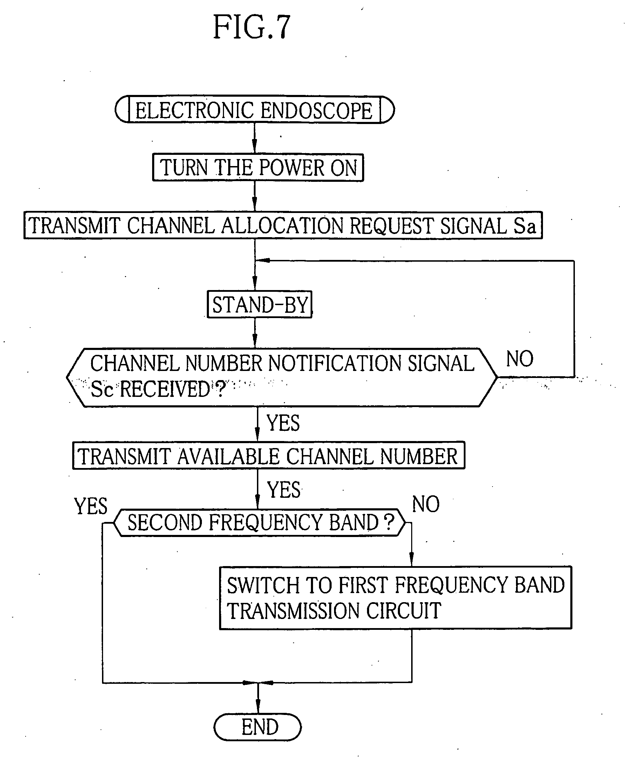

As shown in Fig. 7, when the power of the electronic endoscope 10e is turned on, the channel allocation request signal Sa is transmitted from the first channel detection circuit 43e in the electronic endoscope 10e to the second channel detection circuit 63e in the processor 11e.

When the first channel detection circuit 43e receives the channel number notification signal Sc from the second channel detection circuit 63e, the first channel detection circuit 43e transmits the notified available channel number (in this case, the channel five in the second frequency band) to the CPU 30e and the transmission frequency band switching circuit 42e.

As described above, when there is an available channel in the second frequency band, the transmission frequency band switching circuit 42e is not actuated, and the initially set second frequency band transmission circuit 41e is used. Thus, the endoscopic diagnosis is started by using the notified available channel, that is, in this case, the channel five in the second frequency band.

As described above, since the channels one-five are allocated to the second frequency band, it is possible to use five electronic endoscopes 10a-10e at the same time within the second frequency band. However, when the channel five in the second frequency band is used by the fifth electronic endoscope 10e, and the four other channels in the second frequency band are already used by the electronic endoscopes 10a-10d, there is no available channel left in the second frequency band. To connect the sixth electronic endoscope 10f (not shown), the first channel detection circuit 43f receives the channel number notification signal Sc (in this case, one of the channels one-five in the first frequency band, for instance, the channel one) from the second channel detection circuit 63f and transmits the notified channel number to the CPU 30f and the transmission frequency band switching circuit 42f.

As described above, when the available channel is in the first frequency band, the transmission frequency band switching circuit 42f switches from the second frequency band transmission circuit 41f to the first frequency band transmission circuit 40f. Thus, the notified available channel, that is, the channel one in the first frequency band is used to start the endoscopic diagnosis.

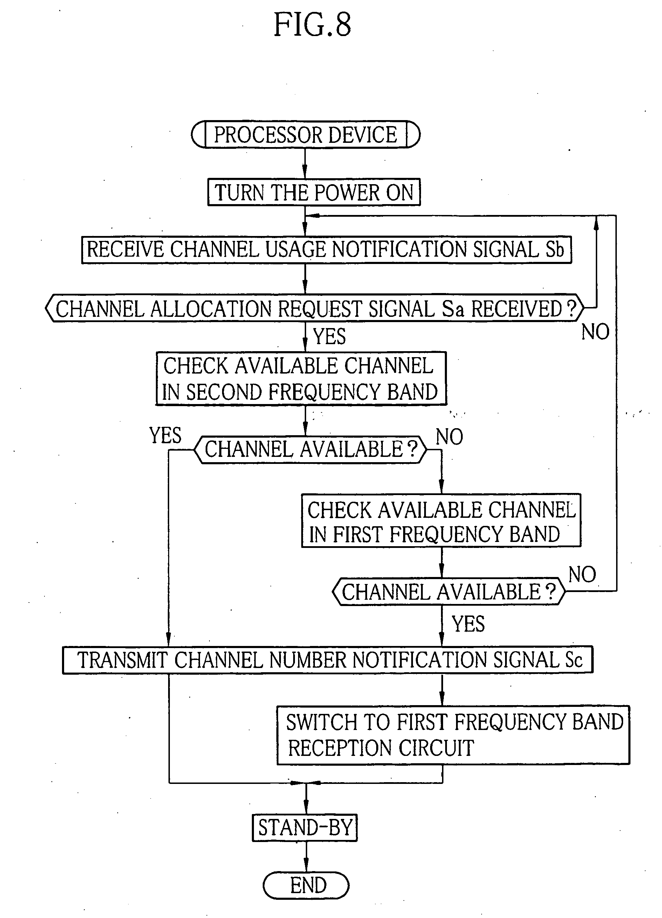

As shown in Fig. 8, in the processor 11e, after the power is turned on, the second channel detection circuit 63e receives the channel-in-use notification signals Sb transmitted at the constant intervals from the first channel detection circuits 43a-43d in the electronic endoscopes 10a-10d (see Fig. 6) . The channel-in-use notification signals Sb notify that the channels one-four in the second frequency band are used. In this state, upon receiving the channel allocation request signal Sa transmitted from the first channel detection circuit 43e of the electronic endoscope 10e, the second channel detection circuit 63e checks the available channel in the second frequency band based on the channel usage condition notified by the channel-in-use notification signals Sb, and detects that the channel five is available.

After the detection of the available channel, the second channel detection circuit 63e transmits the channel number notification signal Sc (in this case, a signal notifying the channel five in the second frequency band is available) to the first channel detection circuit 43e according to the detection result. At this time, the reception frequency band switching circuit 62 is not actuated. The processor 11e uses the notified available channel, that is, the channel five in the second frequency band reception circuit 61e to receive the radio wave 12e from the electronic endoscope 10e.

When the channel five in the second frequency band is used by the fifth electronic endoscope 10e and the four other channels in the second frequency band are already used by the electronic endoscopes 10a-10d, there is no available channel left in the second frequency band according to the channel usage notified by the channel-in-use notification signals Sb transmitted from the above electronic endoscopes 10a-10e. To connect the sixth electronic endoscope 10f, the second channel detection circuit 63f checks the available channel in the first frequency band, and detects that the channels one-five are available.

After the detection of the available channel, the second channel detection circuit 63f transmits the channel number notification signal Sc (in this case, a signal notifying that one of the channels one-five in the first frequency band, for instance, the channel one is available) to the first channel detection circuit 43f according to the above detection results. At this time, the reception frequency band switching circuit 62f switches from the second frequency band reception circuit 61f to the first frequency band reception circuit 60f, and the processor 11f receives the radio wave 12f from the electronic endoscope 10f using the detected available channel, that is, the channel one in the first frequency band.

When ten sets of electronic endoscopes 2a-2j, that is, ten electronic endoscopes 10a-10j and ten processors 11a-11j are provided and currently used for the endoscopic diagnoses, there is no available channel in either of the first and the second frequency bands. In order to use eleventh electronic endoscope 10k, the electronic endoscope 10k remains in a standby state until the examination using one of the electronic endoscopes 10a-10j, for instance, the electronic endoscope 10a is completed and the power of the electronic endoscope 10a is turned off to vacate one channel and the channel number notification signal Sc is received from the second channel detection section 63a. The second channel detection circuit 63a is not able to receive the channel allocation request signal Sa from the first channel detection circuit 43k while the electronic endoscope 10a is used. When the power of the electronic endoscope 10a is turned off, the second channel detection circuit 63a returns to the state for receiving the channel allocation request signal Sa. Upon receiving the channel allocation request signal Sa from the first channel detection circuit 43k, the second channel detection circuit 63a detects that the channel which had been used by the electronic endoscope 10a is now available according to the usage condition notified by the channel-in-use notification signals Sb. Thereby, the second channel detection circuit 63a transmits the available channel number to the first channel detection circuit 43k.

As described above in detail, the electronic endoscope 10 and the processor 11 of the electronic endoscope apparatus 2 transmit and receive signals via the radio waves 12 within predetermined frequency bands to which plural channels are respectively allocated. The electronic endoscope 10 has the transmission frequency band switching circuit 42 which switches between the first and second frequency bands, and the first channel detection circuit 43 for detecting the available channel. The processor 11 has the reception frequency band switching circuit 62 which switches between the first and second frequency bands, and the second channel detection circuit 63 for detecting the available channel. The transmission and reception frequency band switching circuits 42, 62 automatically switch the transmission and reception frequency bands according to detection results of the first and second channel detection circuits 43, 63. Accordingly, the radio interference between the electronic endoscope apparatuses is securely prevented. Further, since it is no longer necessary to manually set the channels and the frequency bands, the operability of the electronic endoscope apparatus is improved.

In the above embodiment, the electronic endoscope 2 for medical uses is described as an example. However, the present invention is not limited to the above, and is also applicable to industrial uses.

Although the present invention has been fully described by the way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An electronic endoscope apparatus (2) constituted of an electronic endoscope (10), for obtaining an image of an observation area of a subject, and a processor (11) corresponding to said electronic endoscope, said electronic endoscope transmitting data of said image to said processor via radio waves,
said electronic endoscope including:
a first channel detection section (43) for detecting an available channel from plural channels previously allocated to each of plural transmission frequency bands of said radio waves; and
a transmission frequency band switching section (42) for automatically switching between said transmission frequency bands according to a detection result of said first channel detection section;
said processor including:
a second channel detection section (63) for detecting an available channel from plural channels previously allocated to each of plural reception frequency bands of said radio waves, and
a reception frequency band switching section (62) for automatically switching between said reception frequency bands according to a detection result of said second channel detection section, wherein said first channel detection section transmits a channel allocation request signal (Sa), for requesting allocation of said available channel, and a channel-in-use notification signal (Sb), for notifying a currently used channel , to said second channel detection section via said radio waves and wherein said second channel detection section transmits a channel number notification signal (Sc) notifying said available channel, based on a detection result of channel usage condition notified by the channel-in-use notification signal (Sb), to said first channel detection section via said radio waves in response
to receiving said channel allocation request signal and wherein said plural transmission frequency bands of said radio waves are multiple of each other and said plural reception frequency bands of said radio waves are multiple of each other.

2. An electronic endoscope apparatus according to claim 1, wherein said first channel detection section transmits a channel allocation request signal (Sa), for requesting allocation of said available channel, to said second channel detection section via said radio waves immediately after a power of said electronic endoscope is turned on.

3. An electronic endoscope apparatus according to claim 1, wherein said first channel detection section transmits said channel-in-use notification signal to said second channel detection section at constant intervals.

4. An electronic endoscope apparatus according to claim 1, wherein said first channel detection section transmits said channel allocation request signal and said channel-in-use notification signal to said second channel detection section by using a different channel from said channels allocated to each of said transmission and reception frequency bands.

5. An electronic endoscope apparatus according to claim 1, wherein said second channel detection section transmits said channel number notification signal to said first channel detection section by using a different channel from said channels allocated to each of said transmission and reception frequency bands.

## Patentansprüche

1. Elektronische Endoskopvorrichtung (2), gebildet aus einem elektronischen Endoskop (10) zum Gewinnen eines Bilds eines Betrachtungsbereichs eines Subjekts, und einem dem elektronischen Endoskop entsprechenden Prozessor (11), wobei das elektronische Endoskop Daten des Bilds über Funkwellen zu dem Prozessor sendet,
wobei das elektronische Endoskop enthält:
einen ersten Kanal-Detektorabschnitt (43) zum Detektieren eines verfügbaren Kanals von mehreren Kanälen, die vorab jedem von mehreren Sendefrequenzbändern der Funkwellen zugeordnet wurden; und
einen Sendefrequenzband-Umschaltabschnitt (52) zum automatischen Umschalten zwischen den Sendefrequenzbändern abhängig von einem Detektierergebnis des ersten Kanal-Detektorabschnitts;
wobei der Prozessor enthält:
einen zweiten Kanal-Detektorabschnitt (63) zum Detektieren eines verfügbaren Kanals von mehreren Kanälen, die vorab jedem von mehreren Empfangsfrequenzbändern der Funkwellen zugeordnet wurden, und
einen Empfangsfrequenzband-Umschaltabschnitt (62) zum automatischen Umschalten zwischen den Empfangsfrequenzbändern abhängig von einem Detektierergebnis des zweiten Kanal-Detektorabschnitts, wobei der erste Kanal-Detektorabschnitt ein Kanalzuordnung-Anforderungssignal (Sa) zum Anfordern der Zuordnung des verfügbaren Kanals und ein Kanal-Belegt-Meldesignal (Sb) zum Melden eines derzeit belegten Kanals über die Funkwellen an den zweiten Kanal-Detektorabschnitt sendet, und wobei der zweite Kanal-Detektorabschnitt ein Kanalnummer-Meldesignal (Sc), das den verfügbaren Kanal meldet, basierend auf einem Detektorergebnis eines Kanalbelegungszustands, der über das Kanal-Belegt-Meldesignal (Sb) gemeldet wird, über die Funkwellen an den ersten Kanal-Detektorabschnitt ansprechend auf den Empfang des Kanalzuordnungs-Anforderungssignals sendet, und wobei die mehreren Sendefrequenzbänder der Funkwellen ein Vielfaches voneinander sind und die mehreren Empfangsfrequenzbänder der Funkwellen ein Vielfaches voneinander sind.

2. Elektronische Endoskopvorrichtung nach Anspruch 1, bei der der erste Kanal-Detektorabschnitt ein Kanalzuordnungs-Anforderungssignal (Sa) zum Anfordern der Zuordnung des verfügbaren Kanals über die Funkwellen an den zweiten Kanal-Detektorabschnitt unmittelbar nach Netz-Einschaltung des elektronischen Endoskops sendet.

3. Elektronische Endoskopvorrichtung nach Anspruch 1, bei der der erste Kanal-Detektorabschnitt das Kanal-Belegt-Meldesignal in konstanten Intervallen an den zweiten Kanal-Detektorabschnitt sendet.

4. Elektronische Endoskopvorrichtung nach Anspruch 1, bei der der erste Kanal-Detektorabschnitt das Kanalzuordnungs-Anforderungssignal und das Kanal-Belegt-Meldesignal unter Verwendung eines von dem jedem der Sende- und Empfangsfrequenzbänder zugeordneten Kanälen verschiedenen Kanal an den zweiten Kanal-Detektorabschnitt sendet.

5. Elektronische Endoskopvorrichtung nach Anspruch 1, bei der der zweite Kanal-Detektorabschnitt das Kanalnummer-Meldesignal an den ersten Kanal-Detektorabschnitt unter Verwendung eines Kanals sendet, der verschieden ist von den Kanälen, die jedem der Sende- und Empfangsfrequenzbänder zugeordnet sind.

## Revendications

1. Appareil formant endoscope électronique (2) composé d'un endoscope électronique (10) pour obtenir une image d'une zone d'observation d'un sujet, et d'un processeur (11) correspondant audit endoscope électronique, ledit endoscope électronique transmettant les données de ladite image audit processeur par d'intermédiaire des ondes radioélectriques, ledit endoscope électronique comprenant :
une première section de détection de canal (43) pour détecter un canal disponible parmi plusieurs canaux préalablement affectés à chacune d'une pluralité de bandes de fréquence de transmission desdites ondes radioélectriques ; et
une section de commutation de bande de fréquence de transmission (42) pour commuter automatiquement entre lesdites bandes de fréquence de transmission selon un résultat de détection de ladite première section de détection de canal ;
ledit processeur comprenant :
une deuxième section de détection de canal (63) pour détecter un canal disponible parmi plusieurs canaux préalablement affectés à chacune parmi une pluralité de bandes de fréquence de réception desdites ondes radioélectriques, et
une section de commutation de bande de fréquence de réception (62) pour commuter automatiquement entre lesdites bandes de fréquence de réception selon un résultat de détection de ladite deuxième section de détection de canal, dans lequel ladite première section de détection de canal transmet un signal de requête d'affectation de canal (Sa), pour demander l'affectation dudit canal disponible, et un signal de notification de canal utilisé (Sb), pour notifier un canal actuellement utilisé, à ladite deuxième section de détection de canal par l'intermédiaire desdites ondes radioélectriques, et dans lequel ladite deuxième section de détection de canal transmet un signal de notification de nombre de canaux (Sc) pour notifier ledit canal disponible, en fonction d'un résultat de détection de condition d'utilisation de canal notifiée par le signal de notification de canal utilisé (Sb), à ladite première section de détection de canal par l'intermédiaire desdites ondes radioélectriques en réponse à la réception dudit signal de requête d'affectation de canal et dans lequel lesdites plusieurs bandes de fréquence de transmission desdites ondes radioélectriques sont des multiples les unes des autres et lesdites plusieurs bandes de fréquence de réception desdites ondes radioélectriques sont des multiples les unes des autres.

2. Appareil formant endoscope électronique selon la revendication 1, dans lequel ladite première section de détection de canal transmet un signal de requête d'affectation de canal (Sa), pour demander l'affectation dudit canal disponible, à ladite deuxième section de détection de canal par l'intermédiaire desdites ondes radioélectriques immédiatement après avoir mis ledit endoscope électronique en marche.

3. Appareil formant endoscope électronique selon la revendication 1, dans lequel ladite première section de détection de canal transmet ledit signal de notification de canal utilisé à ladite deuxième section de détection de canal à intervalles constants.

4. Appareil formant endoscope électronique selon la revendication 1, dans lequel ladite première section de détection de canal transmet ledit signal de requête d'affectation de canal et ledit signal de notification de canal utilisé à ladite deuxième section de détection de canal en utilisant un canal différent desdits canaux affectés à chacune desdites bandes de fréquence de transmission et de réception.

5. Appareil formant endoscope électronique selon la revendication 1, dans lequel ladite deuxième section de détection de canal transmet ledit signal de notification de nombre de canaux à ladite première section de détection de canal en utilisant un canal différent desdits canaux affectés à chacune desdites bandes de fréquence de transmission et de réception.
